# EUROPEAN PATENT APPLICATION

(11) **EP 3 666 932 A1**
(43) Date of publication of application: **17.06.2020**
(21) Application number: 18020636.9
(22) Date of filing: 14.12.2018
(51) Int. Cl.: C25D 11/26, A61L 27/00, B22F 3/105

(54) **PRODUCTION METHOD OF A PERSONALISED, BIOCOMPATIBLE AND BIOACTIVE ORTHOPAEDIC IMPLANT**

(71) Applicant: Nano-implant Sp. z o.o., 87-100 Torun (PL)
(72) Inventor: Zdrojewski, Andrzej, 86-300 Grudzi?dz (PL); Piszczek, Piotr, 87-100 Toru? (PL); Radtke, Aleksandra, 87-100 Toru? (PL)
(74) Representative: Cwiklinski, Grzegorz

(57) **Abstract**

The production method of the implant made of Ti6A14V titanium alloy with a coating made of titanium dioxide nanotubes includes the creation of a virtual implant model on the basis of computer tomography images of the anatomical shape of the bone from before a fracture and the adjustment of the shape of the implant to the anatomical shape of the bone, and then creation in the process of selective laser sintering of Ti6A14V titanium alloy powder in 3D printing technology, and then a thermal stress relieving and mechanical preparation of the surface by a sandblasting method. Thereafter, the surface is degreased favourably in ethyl alcohol with the use of an ultrasonic cleaner and washed in distilled water also with the use of an ultrasonic cleaner, then the implant is immersed for 20-40 seconds in a mix of the hydrofluoric acid pure for analysis (p.a.), nitric acid (V) (p.a.) and distilled water, mixed in a 1:4:5 to 2:3:6 ratio. Then, a process of anodic oxidation of the implant surface is carried out in 0.3% solution of the hydrofluoric acid (p.a.) for 15-25 minutes, at a voltage of 5 V to 8V with the implant as an anode with a distance of 1.5 to 2.5 cm between a platinum plate as a cathode. After the anodic oxidation process, the implant is washed in distilled water by means of an ultrasonic cleaner with the addition of Al₂O₃ powder whose grain sizes are equal to 50 nm and then it is washed in acetone (p.a.) also with the use of an ultrasonic cleaner and dried in an argon beam and then it is held in a dryer at a temperature of 393 K. Titanium dioxide nanotubes of 100-150 nm thick and nanotube diameter of 18 to 25 nm are created on the surface of the implant.

## Description

The subject matter of the invention is a production method of a biocompatible and bioactive orthopaedic implant intended particularly for orthopaedic surgery.

Orthopaedic implants made of titanium alloy Ti6A14V are commonly known. This alloy, compared with other metallic materials, is characterised by good biocompatibility, good corrosion resistance, the lowest Young's modulus, a high relative strength and a low specific weight.

An inconvenience of previously applied orthopaedic implants made of Ti6Al4V alloy is the lack of antibacterial activity of this material which resulted in the fact that often shortly after implant insertion to the recipient's body, they were rejected by the recipient's body. Sometimes postsurgical complications related to inflammation and the weakening general resistance of the patient's body occur even after a longer period.

Orthopaedic implants made of Ti6Al4V alloy anatomically adapted to patient's needs are well-known. This method consists in the creation of a virtual model of a fragment of the bone structure on the basis of computer tomography images, processing it to a form necessary for surgical intervention and then in the creation of an implant with the use of the technology of selective laser sintering of Ti6Al4V titanium alloy powder.

The implant prepared in such a manner is inserted in the patient's body for the purpose of the reconstruction or stabilisation of the bone system. It is also important to choose a system of fixings and the stabilisation of the bone in relation to the implant for each type of intervention. In the case of a fracture reconstruction, it is necessary to recreate the angular and spatial bone system as to ensure that the system will be similar to the one from before the fracture after the bone tissue regeneration. Despite a perfect adjustment of the implant in terms of its size and shape, its inconvenience is still a low biocompatibility and a lack of antibacterial activity.

The mechanical and physical methods of the modification of metallic biomaterials surface, including titanium alloy Ti6Al4V are well-known. These methods include machining, polishing, sandblasting, Physical Vapour Deposition, ion-beam sputtering, vaporisation and plasma discharge. Their main purpose is to obtain an implant surface with a defined topography and roughness and to obtain at the same time a surface presenting a better adhesion in bonding with the bone.

An inconvenience of the application of the mechanical and physical method of modification of metallic bio-materials surface, including the Ti6Al4V titanium alloy is that despite the control of porosity of the coating having impact on the improvement of the osseointegration properties of the implant, they have no influence on its anti-inflammatory and antibacterial properties.

The chemical methods of the modification of metallic bio-material substrate, such as Ti6Al4V alloy, are well-known. These methods may include chemical reactions in acidic or basic environment or at the presence of hydrogen peroxide, the sol-gel method, chemical vapour deposition and electrochemical oxidation applying voltages higher than 10 V. All these methods lead to the creation of a layer of titanium dioxide with a different structure and morphology on the surface of the implant thereby providing the original surface of the Ti6Al4V alloy a better biocompatibility and bioactivity.

An inconvenience of the application of chemical and electrochemical method of modification of metallic bio-materials surface, including the titanium alloy Ti6Al4V, is that despite the possibility to control morphology of the coating's surface having an influence on the improvement of osseointegration properties of the implant, they have no influence on the modification of its anti-inflammatory and antibacterial properties.

The production method of personalised, biocompatible and bioactive orthopaedic implant according to the invention consists in the creation of a virtual model of the implant on the basis of computed tomography images of the anatomical shape of the bone from before the fracture and adjustment of the implant shape to the anatomical shape of the bone and then in creation in the process of selective laser sintering of Ti6Al4V titanium alloy powder in 3D printing technology, and then in a thermal stress relieving and mechanical preparation of the surface by sandblasting method. Then, the surface is degreased favourably in ethyl alcohol with the use of an ultrasonic cleaner and washed in distilled water also with the use of ultrasonic cleaner, after which the implant is immersed for 20-40 seconds in a mix of the hydrofluoric acid pure for analysis (p.a.), nitric acid (V) (p.a.) and distilled water mixed at 1:4:5 to 2:3:6 ratio. Then, a process of anodic oxidation of the implant surface is carried out in 0.3% solution of the hydrofluoric acid (p.a.) for 15-25 minutes, at a voltage from 5 V to 8V with the implant as an anode with a distance from 1.5 to 2.5 cm between a platinum plate as a cathode. After the anodic oxidation process, the implant is washed in distilled water by means of an ultrasonic cleaner with the addition of Al₂O₃ powder whose grain sizes are equal to 50 nm, and then it is washed in acetone (p.a.) also with the use of an ultrasonic cleaner and dried in argon beam and then it is held in a dryer at a temperature of 393 K. A titanium dioxide nanotubes of 100-150 nm thick and nanotubes diameter from 18 to 25 nm are created on the surface of the implant.

It is advantageous when silver nanograins are applied on the implant surface with the use of the chemical vapour deposition (CVD) method, by applying silver pentafluoropropionate trihydrate [Ag₅(OOCC2F₅)₅(H₂O)₃], as a precursor through the placement of the implant dried in an argon beam in a reactor, after which the pressure inside the reactor is lowered to p = 8.0·10⁻² hPa and, simultaneously, the heating of the reactor is switched on, leading the temperature of the deposition zone (TD) to 565 K and, after 30 minutes, an argon flow of the flow rate of 0.23 cm³ min⁻¹ is switched on and the total pressure of the reactor is set at the level of p = 5.0·10⁻¹ hPa, after which a sublimation oven is switched on, setting the temperature of this part of the reactor at the level of Tᵥ = 508 K and the deposition process is carried out for 30 minutes. Thereafter, the reactor is cooled to the room temperature, obtaining an implant the surface of which is composed of a layer of titanium dioxide nanotubes of approx. 150 nm thick containing 1.5%-2% by weight dispersed silver nanograins.

The reflection of the bone shape causes better adhesion of the implant and reduces the risk of pressure of the bone causing an increased probability of inflammation. The titanium dioxide nanotubes layer with a strictly defined structure and morphology is intended to improve the process of integration of the implant with the bone and to prevent the creation of inflammation shortly after implant insertion in the recipient's body. The enrichment of the titanium dioxide nanotubes coating with metallic silver nanoparticles is intended to prevent the creation of bacterial biofilm on the implant surface for a longer time after the implant insertion in the recipient's body and, at the same time, to have antimicrobial (antibacterial and antimycotic) effects.

The subject matter of the invention is further illustrated by examples of its execution without limiting the scope of its protection.

### Example I.

Production method of a personalised implant of a radius volar plate made of Ti6Al4V titanium alloy optimised in terms of the biocompatibility and anti-inflammatory properties through a modified implant surface that is created by a titanium dioxide nanotubes layer. The implant is applied in orthopaedics for connection after a bone fracture for the need of an individual patient.

The stage 1 A virtual model of the radius volar plate implant was made on the basis of computed tomography images, adjusting the shape of the implant to the anatomical shape of the bone from before the fracture that presents a virtual model of the implant along with the holes to introduce anchor bolts located in the most optimal places developed on the basis of computed tomography images.

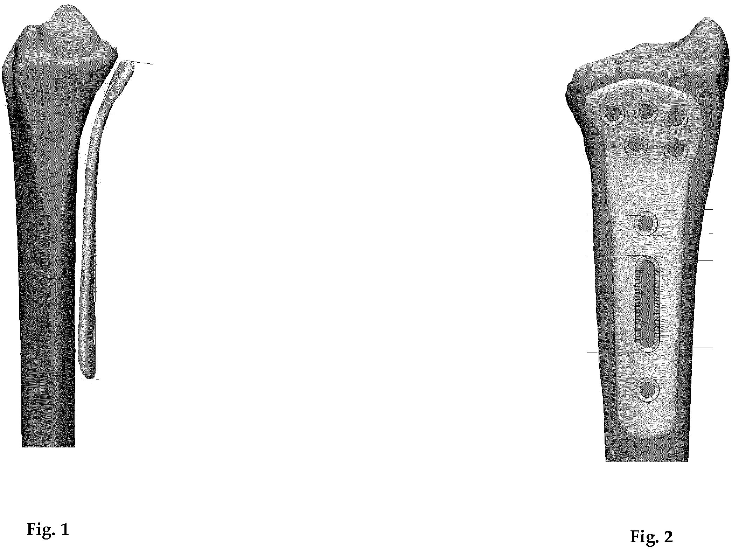

The virtual model of the implant along with the holes to introduce anchor bolts located in the most optimal places was made with the use of the selective laser sintering technology of Ti6Al4V titanium alloy powder. The radius plate implant adapted to the anatomical shape of the bone from before the fracture was illustrated on Fig. 2.

After the printing process, the implant was subjected to thermal stress relieving in order to eliminate internal stress and treated mechanically by means of sandblasting technology.

Stage 2 The radius plate implant surface made of Ti6Al4V alloy created in laser sintering technology was degreased by immersion in 70% ethyl alcohol for 5 minutes with the use of an ultrasonic cleaner and washed for 5 minutes in distilled water with the reuse of an ultrasonic cleaner. The implant, after being removed from the ultrasonic cleaner, was immersed for 30 seconds in a mix of the hydrofluoric acid (p.a.), nitric acid(V) (p.a.) and distilled water, mixed in a 1:4:5 ratio.

Stage 3 The anodic oxidation process of the implant is carried out for 20 minutes at a voltage of 5 V in a 0.3% hydrofluoric acid solution (p.a.) thereby fulfilling the role of an electrolyte. The distance between the implant as an anode and the platinum plate as a cathode is 2 cm. After the anodic oxidation process, the implant is washed in distilled water by means of an ultrasonic cleaner with the addition of Al₂O₃ powder whose grain sizes are equal to 50 nm, and then it is also washed in acetone (p.a.) with the use of an ultrasonic cleaner and dried in argon beam and then it is held in a dryer at a temperature of 393 K. All operations related to the anodic oxidation process lead to the creation of a layer of titanium dioxide nanotubes of 100-150 nm thick and nanotube diameter of 18 to 25 nm on the radius plate implant surface made in the selective laser sintering of Ti6Al4V alloy powder technology.

### Example II.

The production method of a personalised implant of a radius volar plate made of Ti6Al4V titanium alloy optimised in terms of the biocompatibility and anti-inflammatory properties through a modified implant surface that is created by a titanium dioxide nanotubes layer containing 1.5%-2% by weight nanotubes of metallic silver. The implant is applied in orthopaedics for connection after a bone fracture for the need of an individual patient.

Stage 1 A virtual model of the radius volar plate implant was made on the basis of computed tomography images, adjusting the shape of the implant to the anatomical shape of the bone from before the fracture (Figure 1). The virtual model of the implant along with the holes to introduce anchor bolts located in the most optimal places was made with the use of selective laser sintering technology of titanium powder or Ti6Al4V titanium alloy. The radius plate implant adapted to anatomical shape of the bone from before the fracture is shown in Fig. 2. After the printing process, the implant was subjected to thermal stress relieving in order to eliminate internal stress and treated mechanically by means of sandblasting technology.

Stage 2 The radius plate implant surface made of Ti6Al4V alloy created in laser sintering technology was degreased by immersion in 70% ethyl alcohol for 5 minutes with the use of an ultrasonic cleaner and washed for 5 minutes in distilled water with of the reuse of an ultrasonic cleaner. The implant, after being removed from the ultrasonic cleaner, was immersed for 30 seconds in a mix of the hydrofluoric acid (p.a.), nitric acid(V) (p.a.) and distilled water, mixed in a 1:4:5 ratio.

Stage 3 The anodic oxidation process of the implant is carried out for 20 minutes at a voltage of 8 V in a 0.3% hydrofluoric acid solution (p.a.) fulfilling the role of an electrolyte. The distance between the implant as an anode and the platinum plate as a cathode is 2 cm. After the anodic oxidation process, the implant is washed in distilled water by means of an ultrasonic cleaner with addition of Al₂O₃ powder whose grain sizes are equal to 50 nm, and then it is washed in acetone (p.a.) also with the use of an ultrasonic cleaner and dried in an argon beam. All operations related to the anodic oxidation process lead to the creation of a layer of titanium dioxide nanotubes 100-150 nm thick and nanotubes if a diameter of 20 to 30 nm on the radius plate implant surface made in the selective laser sintering of Ti6Al4V alloy powder technology.

Stage 4 The surface of the implant made in 3D technology constituted by a layer of titanium dioxide nanotubes was enriched with silver nanotubes by the chemical vapour deposition (CVD) method, applying the silver pentafluoropropionate trihydrate [Ag₅(OOCC₂F₅)₅(H₂O)₃] as a precursor. The process of the creation of silver nanograins is characterised in that 100 mg of the silver pentafluoropropionate trihydrate is put in the sublimation vessel of the CVD reactor, whereas the implant dried in an argon beam is placed in the reactor deposition zone. The reactor is closed and the pressure inside the reactor is reduced to p = 8.0·10⁻² hPa and, at the same time, the heating of the reactor is switched on, leading the temperature of the deposition zone (TD) to 565 K. After 30 minutes, the argon flow of flow rate of 0.23 cm³ min⁻¹ is switched on, and the total pressure of the reactor is set at the level of p = 5.0·10⁻¹ hPa. After the stabilisation of the working condition, the sublimation oven is switched on, setting the temperature of this part of the reactor at a level of Tᵥ = 508 K. the deposition process is carried out for 30 minutes. Thereafter, the reactor is cooled to room temperature, obtaining an implant the surface of which is composed of a layer of titanium dioxide nanotubes containing 1.5%-2% by weight dispersed silver nanograins.

The crystal system of the obtained layers of titanium dioxide is determined on the basis of a Raman spectroscopy test. The tests indicated that the layers of titanium dioxide nanotubes made in the process of anodising of the implant surface are amorphic. The cubic system of silver nanograins was identified by means of the X-ray diffraction method based on the following lines: 2*Θ* = 38.2° (111), 44.3° (200), 64.5° (220) and 77.5° (311).

The Figure shows the view of a radius plate implant adjusted to the anatomical shape of the bone that was created with the use of technology of selective laser sintering of Ti6Al4V titanium alloy powder.
**Fig. 1** shows a side view of the virtual implant model of a radius plate adjusted to the bone by its shape along with the holes for the introduction of anchor bolts in the most optimal places.
**Fig. 2** shows a top view of the virtual implant model of a radius plate adjusted to the bone by its shape along with the holes for the introduction of anchor bolts in the most optimal places.

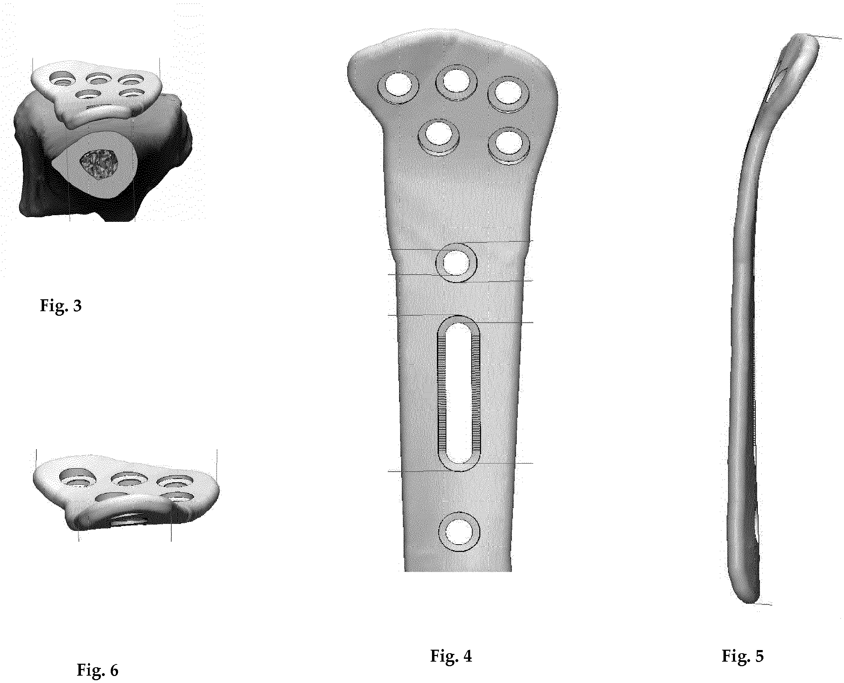

**Fig. 3** shows a front view of the virtual implant model of a radius plate adjusted to the bone by its shape along with the holes for the introduction of anchor bolts in the most optimal places.
**Fig. 4** shows a top view of the radius plate implant adjusted to the anatomical shape of the bone.
**Fig. 5** shows a side view of the radius plate implant adjusted to the anatomical shape of the bone.
**Fig. 6** shows a front view of the radius plate implant adjusted to the anatomical shape of the bone.

## Claims

1. The production method of the implant made of Ti6A14V titanium alloy with a coating made of titanium dioxide nanotubes is **characterised in that** it consists in the creation of a virtual implant model on the basis of computer tomography images of the anatomical shape of the bone from before a fracture and the adjustment of the shape of the implant to the anatomical shape of the bone, and then creation in the process of selective laser sintering of Ti6Al4V titanium alloy powder in 3D printing technology, and then a thermal stress relieving and mechanical preparation of the surface by a sandblasting method. Thereafter, the surface is degreased favourably in ethyl alcohol with the use of an ultrasonic cleaner and washed in distilled water also with the use of an ultrasonic cleaner, then the implant is immersed for 20-40 seconds in a mix of the hydrofluoric acid pure for analysis (p.a.), nitric acid (V) (p.a.) and distilled water, mixed in a 1:4:5 to 2:3:6 ratio. Then, a process of anodic oxidation of the implant surface is carried out in 0.3% solution of the hydrofluoric acid (p.a.) for 15-25 minutes, at a voltage of 5 V to 8V with the implant as an anode with a distance of 1.5 to 2.5 cm between a platinum plate as a cathode. After the anodic oxidation process, the implant is washed in distilled water by means of an ultrasonic cleaner with the addition of Al₂O₃ powder whose grain sizes are equal to 50 nm and then it is washed in acetone (p.a.) also with the use of an ultrasonic cleaner and dried in an argon beam and then it is held in a dryer at a temperature of 393 K. Titanium dioxide nanotubes of 100-150 nm thick and nanotube diameter of 18 to 25 nm are created on the surface of the implant.

2. The method according to claim 1 **characterised in that** silver nanograins are applied on the implant surface by means of the chemical vapour deposition (CVD) method, applying silver pentafluoropropionate trihydrate [Ag₅(OOCC₂F₅)₅(H₂O)₃], as a precursor through the placement of the implant dried in an argon beam in a reactor, after which the pressure inside the reactor is lowered to p = 8.0·10⁻² hPa and, simultaneously, the heating of the reactor is switched on, leading the temperature of the deposition zone (TD) to 565 K and after 30 minutes, an argon flow of the flow rate of 0.23 cm³ min⁻¹ is switched on and the total pressure of the reactor is set at the level of p = 5.0·10⁻¹ hPa, after which a sublimation oven is switched on, setting the temperature of this part of the reactor at the level of Tv = 508 K and the deposition process is carried out for 30 minutes. Thereafter, the reactor is cooled to room temperature, obtaining an implant the surface of which is composed of a layer of titanium dioxide nanotubes of approx. 150 nm thick containing 1.5%-2% by weight dispersed silver nanograins.

3. The method according to the claim is **characterised in that** the anodic oxidation process is carried out using an electrochemical system in which the distance between the platinum plate which fulfils a role of cathode and the modified implant that acts as an anode is equal to 2 cm.

4. The method according to claim 3 **is characterised in that** the anodic oxidation process lasts 20 minutes.

5. The method according to claim 3 **is characterised in that** the anodic oxidation process is carried out at a voltage of 5 V.

6. The method according to claim 3 **is characterised in that** the anodic oxidation process is carried out at a voltage of 8 V.

7. The method according to claim 13, the 3D implant surface layer enrichment process with sliver nanograins, is carried out at a total pressure equal to 5.0·10⁻¹ hPa.

8. The method according to claim 13 **is characterised in that** the 3D implant surface layer enrichment process with sliver nanograins is carried out for 30 minutes.

9. The method according to claim 13 **is characterised in that** the 3D implant surface layer enrichment process with sliver nanograins is carried out with the use of silver pentafluoropropionate trihydrate [Ag₅(OOCC₂F₅)₅(H₂O)₃] as a precursor at the amount of 100 mg per process.

10. The method according to claim 13 **is characterised in that** the 3D implant surface layer enrichment process with sliver nanograins is carried out at a deposition temperature equal to 565 K and a precursor evaporation temperature at the level of 508 K.
